# EUROPEAN PATENT SPECIFICATION

(11) **EP 2 351 857 B1**
(45) Date of publication and mention of the grant of the patent: **29.10.2014**
(21) Application number: 10190717.8
(22) Date of filing: 14.02.2006
(51) Int. Cl.: C12Q 1/68

(54) **Compositions and methods for detecting pathogen specific nucleic acids in urine**
Zusammensetzungen und Verfahren zum Nachweis von für Krankheitserreger spezifischen Nukleinsäuren in Urin
Compositions et méthodes de détection d'acides nucléiques pathogènes spécifiques dans l'urine

(30) Priority: 17.02.2005 IT RM20050067; 25.05.2005 US 137934; 16.06.2005 US 691186 P; 10.02.2006 US 351799
(43) Date of publication of application: 03.08.2011
(62) Divisional of application: 06735068.6
(73) Proprietor: TrovaGene, Inc., San Diego, CA 92121 (US)
(72) Inventor: Milkonyan, Hovsep, Princeton, NJ 08540 (US); Cannas, Angela, I-09031, Arbus (IT); Tomei, Louis, David, I-00030, Rome (IT); Umansky, Samuil, R., Princeton, NJ 08540 (US)
(74) Representative: Roques, Sarah Elizabeth

(56) References cited:
- EP-A1- 1 347 060
- WO-A2-02/04681
- SECHI L A ET AL: "Detection of Mycobacterium tuberculosis by PCR analysis of urine and other clinical samples from AIDS and non-HIV-infected patients", MOLECULAR AND CELLULAR PROBES 199708 GB LNKD- DOI:10.1006/MCPR.1997.0119, vol. 11, no. 4, August 1997 (1997-08), pages 281-285, XP002639921, ISSN: 0890-8508
- ACETI A ET AL: "Identification of HIV patients with active pulmonary tuberculosis using urine based polymerase chain reaction assay", THORAX 1999 GB, vol. 54, no. 2, 1999, pages 145-146, XP002639922, ISSN: 0040-6376
- KAFWABULULA M L ET AL: "Evaluation of PCR-based methods for the diagnosis of tuberculosis by identification of mycobacterial DNA in urine samples", INTERNATIONAL JOURNAL OF TUBERCULOSIS AND LUNG DISEASE 2002 FR, vol. 6, no. 8, 2002, pages 732-737, XP009148974, ISSN: 1027-3719
- TORREA G ET AL: "PCR-based detection of the Mycobacterium tuberculosis complex in urine of HIV-infected and uninfected pulmonary and extrapulmonary tuberculosis patients in Burkina Faso", JOURNAL OF MEDICAL MICROBIOLOGY 200501 GB LNKD- DOI:10.1099/JMM.0.45688-0, vol. 54, no. 1, January 2005 (2005-01), pages 39-44, XP002639923, ISSN: 0022-2615
- HEMAL A K ET AL: "Polymerase chain reaction in clinically suspected genitourinary tuberculosis: comparison with intravenous urography, bladder biopsy, and urine acid fast bacilli culture", UROLOGY 200010 US LNKD- DOI:10.1016/S0090-4295(00)00668-3, vol. 56, no. 4, October 2000 (2000-10), pages 570-574, XP002639924, ISSN: 0090-4295
- DATABASE EMBL [Online] 22 February 2008 (2008-02-22), "METHOD FOR SIMULTANEOUS DETECTION OF TUBERCLE BACILLUS(TB) ANDNONTURBERCULOUS MYCOBACTERIA(NTM) BY MULTIPLEX-NESTED POLYMERASECHAIN REACTION.", XP002639925, retrieved from EBI accession no. EM_PAT:DI036880 Database accession no. DI036880 & KR 2003 0075315 A (BIOCORE CO LTD [KR]) 26 September 2003 (2003-09-26)

### SEQUENCE LISTING

<110> Instituto Nazionale per le Malattie Infettive IRCCS Lazzaro Spallanzani Melkonyan, Hovsep Cannas, Angela Tomei, Louis David Umansky, Samuil R.
<120> COMPOSITIONS AND METHODS FOR DETECTING PATHOGEN SPECIFIC NUCLEIC ACIDS IN URINE
<130> 29480-503-061
<140> PCT/US2006/005225
   <141> 2006-02-14
<150> RM2005A000068
   <151> 2005-02-17
<150> US 11/137,934
   <151> 2005-05-25
<150> 60/691,186
   <151> 2005-06-16
<160> 51
<170> PatentIn version 3.2
<210> 1
   <211> 22
   <212> DNA
   <213> Artificial sequence
<220>
   <223> Chemically synthesized primer
<400> 1
   aagacagtgt tgtgggtgta gg 22
<210> 2
   <211> 18
   <212> DNA
   <213> Artificial sequence
<220>
   <223> Chemically synthesized primer
<400> 2
   tccaaggccg ctttacac 18
<210> 3
   <211> 20
   <212> DNA
   <213> Artificial sequence
<220>
   <223> Chemically synthesized primer
<400> 3
   ggcacaggtt tcatccattc 20
<210> 4
   <211> 25
   <212> DNA
   <213> Artificial sequence
<220>
   <223> Chemically synthesized primer
<400> 4
   gataggtctc tctttatgtc ttcag 25
<210> 5
   <211> 25
   <212> DNA
   <213> Artificial sequence
<220>
   <223> chemically synthesized primer
<400> 5
   tcrgaaattt gggrmtcagc gttac 25
<210> 6
   <211> 30
   <212> DNA
   <213> Artificial sequence
<220>
   <223> Chemically synthesized primer
<400> 6
   tccataaaat ttyggatbbd tygggtgttg 30
<210> 7
   <211> 22
   <212> DNA
   <213> Artificial sequence
<220>
   <223> Chemically synthesized primer
<400> 7
   acggatcdtt ttgawgggac ac 22
<210> 8
   <211> 29
   <212> DNA
   <213> Artificial sequence
<220>
   <223> Chemically synthesized primer
<400> 8
   agcdycayac crcaavaaag tcatgcygc 29
<210> 9
   <211> 19
   <212> DNA
   <213> Artificial sequence
<220>
   <223> Chemically synthesized primer
<400> 9
   ctgadaccgt tcctacagc 19
<210> 10
   <211> 24
   <212> DNA
   <213> Artificial sequence
<220>
   <223> Chemically synthesized primer
<400> 10
   yttyacracc gtgatyattc cagc 24
<210> 11
   <211> 28
   <212> DNA
   <213> Artificial sequence
<220>
   <223> Chemically synthesized primer
<400> 11
   ckccaaayga tcccatacac agcgagag 28
<210> 12
   <211> 23
   <212> DNA
   <213> Artificial sequence
<220>
   <223> Chemically synthesized primer
<400> 12
   tgatgggacy aaactcgtaa ccg 23
<210> 13
   <211> 29
   <212> DNA
   <213> Artificial sequence
<220>
   <223> Chemically synthesized primer
<400> 13
   cttcattttt taagaacaac tcaccagga 29
<210> 14
   <211> 25
   <212> DNA
   <213> Artificial sequence
<220>
   <223> Chemically synthesized primer
<400> 14
   accattrgcc tcaatagggg tatgc 25
<210> 15
   <211> 23
   <212> DNA
   <213> Artificial sequence
<220>
   <223> Chemically synthesized primer
<400> 15
   cactgggcgt aaagagygcg tag 23
<210> 16
   <211> 21
   <212> DNA
   <213> Artificial sequence
<220>
   <223> Chemically synthesized primer
<400> 16
   ccacctrcct ctcccayact c 21
<210> 17
   <211> 28
   <212> DNA
   <213> Artificial sequence
<220>
   <223> Chemically synthesized primer
<400> 17
   ggttaagcca taggatttca cayctgac 28
<210> 18
   <211> 26
   <212> DNA
   <213> Artificial sequence
<220>
   <223> Chemically synthesized primer
<400> 18
   gggtattggc ctaacatggc tatgac 26
<210> 19
   <211> 20
   <212> DNA
   <213> Artificial sequence
<220>
   <223> Chemically synthesized primer
<400> 19
   aggctccctc tccggaatcg 20
<210> 20
   <211> 23
   <212> DNA
   <213> Artificial sequence
<220>
   <223> Chemically synthesized primer
<400> 20
   agaattgggt aatttacgcg cct 23
<210> 21
   <211> 23
   <212> DNA
   <213> Artificial sequence
<220>
   <223> Chemically synthesized primer
<400> 21
   cttgatttct tggatggtga tgc 23
<210> 22
   <211> 24
   <212> DNA
   <213> Artificial sequence
<220>
   <223> chemically synthesized primer
<400> 22
   ggttaagatc tcgttcgtta tcgg 24
<210> 23
   <211> 22
   <212> DNA
   <213> Artificial sequence
<220>
   <223> Chemically synthesized primer
<400> 23
   cggcttaatt tgactcaaca cg 22
<210> 24
   <211> 17
   <212> DNA
   <213> Artificial sequence
<220>
   <223> chemically synthesized primer
<400> 24
   agtttyccgc cccggag 17
<210> 25
   <211> 19
   <212> DNA
   <213> Artificial sequence
<220>
   <223> Chemically synthesized primer
<400> 25
   cccagtttbc cgccycgga 19
<210> 26
   <211> 24
   <212> DNA
   <213> Artificial sequence
<220>
   <223> Chemically synthesized primer
<400> 26
   ctattggaga ttatggagct gtgc 24
<210> 27
   <211> 21
   <212> DNA
   <213> Artificial sequence
<220>
   <223> Chemically synthesized primer
<400> 27
   cacacaccga accgargttg c 21
<210> 28
   <211> 21
   <212> DNA
   <213> Artificial sequence
<220>
   <223> Chemically synthesized primer
<400> 28
   gggagaacgt actggggcgt c 21
<210> 29
   <211> 21
   <212> DNA
   <213> Artificial sequence
<220>
   <223> Chemically synthesized primer
<400> 29
   tcgctgtagt tcgtcttggt g 21
<210> 30
   <211> 24
   <212> DNA
   <213> Artificial sequence
<220>
   <223> Chemically synthesized primer
<400> 30
   ctgttgctgt taaagggttc gtag 24
<210> 31
   <211> 21
   <212> DNA
   <213> Artificial sequence
<220>
   <223> Chemically synthesized primer
<400> 31
   tccgacatgg acgggacgac c 21
<210> 32
   <211> 18
   <212> DNA
   <213> Artificial sequence
<220>
   <223> Chemically synthesized primer
<300>
   <301> A Belli et al. <302> Simplified polymerase chain reaction detection of new world Leishmania in clinical specimens of cutaneous Leishmaniasis <303> Am. J. Trop. Med. Hyg. <304> 58 <305> 1 <306> 102-109 <307> 1998
<400> 32
   gtgggggagg ggcgttct 18
<210> 33
   <211> 21
   <212> DNA
   <213> Artificial sequence
<220>
   <223> Chemically synthesized primer
<300>
   <301> A Belli et al. <302> Simplified polymerase chain reaction detection of new world Leishmania in clinical specimens of cutaneous Leishmaniasis <303> Am. J. Trop. Med. Hyg. <304> 58 <305> 1 <306> 102-109 <307> 1998
<400> 33
   attttacacc aacccccagt t 21
<210> 34
   <211> 22
   <212> DNA
   <213> Artificial sequence
<220>
   <223> Chemically synthesized primer
<400> 34
   accagcacct aaccggctgt gg 22
<210> 35
   <211> 22
   <212> DNA
   <213> Artificial sequence
<220>
   <223> Chemically synthesized primer
<400> 35
   catcgtggaa gcgacccgcc ag 22
<210> 36
   <211> 22
   <212> DNA
   <213> Artificial sequence
<220>
   <223> Chemically synthesized primer
<400> 36
   gtaggcgaac cctgcccagg tc 22
<210> 37
   <211> 19
   <212> DNA
   <213> Artificial sequence
<220>
   <223> Chemically synthesized primer
<400> 37
   gccccatcga cctactacg 19
<210> 38
   <211> 20
   <212> DNA
   <213> Artificial sequence
<220>
   <223> Chemically synthesized primer
<400> 38
   tgaggtctgc tacccacagc 20
<210> 39
   <211> 21
   <212> DNA
   <213> Artificial sequence
<220>
   <223> Chemically synthesized primer
<400> 39
   ccctgaaccg tgagggcatc g 21
<210> 40
   <211> 23
   <212> DNA
   <213> Artificial sequence
<220>
   <223> chemically synthesized primer
<400> 40
   acaggccgag tttggtcatc agc 23
<210> 41
   <211> 20
   <212> DNA
   <213> Artificial sequence
<220>
   <223> Chemically synthesized primer
<400> 41
   ggttatatgt tccagaatcc 20
<210> 42
   <211> 20
   <212> DNA
   <213> Artificial sequence
<220>
   <223> Chemically synthesized primer
<400> 42
   atatccagca taatcatcca 20
<210> 43
   <211> 29
   <212> DNA
   <213> Artificial sequence
<220>
   <223> Chemically synthesized primer
<400> 43
   aaggtgtaga attaaggaat gatagtgag 29
<210> 44
   <211> 23
   <212> DNA
   <213> Artificial sequence
<220>
   <223> Chemically synthesized primer
<400> 44
   tccagcataa tcatccacag cat 23
<210> 45
   <211> 23
   <212> DNA
   <213> Artificial sequence
<220>
   <223> Chemically synthesized primer
<400> 45
   aacaacgaag tacaatacaa gac 23
<210> 46
   <211> 19
   <212> DNA
   <213> Artificial sequence
<220>
   <223> Chemically synthesized primer
<400> 46
   gctgttaacg gcttcaaga 19
<210> 47
   <211> 24
   <212> DNA
   <213> Artificial sequence
<220>
   <223> Chemically synthesized primer
<400> 47
   aattggagaa atatagaagt gatg 24
<210> 48
   <211> 21
   <212> DNA
   <213> Artificial sequence
<220>
   <223> Chemically synthesized primer
<400> 48
   aagtacaagt gctggaccta c 21
<210> 49
   <211> 25
   <212> DNA
   <213> Artificial sequence
<220>
   <223> Chemically synthesized primer
<400> 49
   caaccgtata tccttctacc tctaa 25
<210> 50
   <211> 21
   <212> DNA
   <213> Artificial sequence
<220>
   <223> Chemically synthesized primer
<400> 50
   ttccagaccg tgacaatgat g 21
<210> 51
   <211> 17
   <212> DNA
   <213> Artificial sequence
<220>
   <223> Chemically synthesized primer
<400> 51
   rggtaggggc gttctgc 17

## Description

### BACKGROUND OF THE INVENTION

There are currently three types of *in vitro* diagnostic systems widely used for the detection of non-viral pathogens. These are direct culture of the pathogenic agent from the biological sample; immunological assays based on the detection of products or antigens of the infectious agent; and indirect immunological assays that can detect antibodies produced against the infectious agent during infection.

In the first system, the principal disadvantage is that the biological sample must be considered to be at risk for the transmission of the pathogenic agent. In the second and third systems, the disadvantages include sample retrieval that is often invasive and potentially infective sample when collected. In the third system, one major disadvantage is that there is often little possibility of discriminating between past and current infections.

More recently, molecular diagnostic methods have been developed based on the detection of the nucleic acids of the pathogenic agent in the blood or plasma samples, or in the cell cultures, taken from the patient. These assays are generally much more sensitive than the immunological assays. However, they may require the presence of special equipment and qualified personnel. Furthermore, the biological samples - in the case of plasma, blood, or cell cultures - are difficult to store unaltered, except under controlled temperature conditions, and are considered to be biohazardous to personnel who handle them.

Recently, molecular diagnostic methods based on transrenal DNA (Tr-DNA) have been described for monitoring the progress of allogeneic transplants, to diagnose the sex of a fetus, and to detect the presence of tumor markers. (Botezatu et al. Clinical Chemistry 46(8):1078-84 (2000); Su et al. Ann. NY Acad. Sci. 1022:81-89 (2004)) For example, U.S. Patent No. 6,251,638 describes an analytical method for detecting male fetal DNA in the urine of pregnant women. U.S. Patent No. 6,287,820 describes a system aimed at the diagnosis of tumors, particularly of adenocarcinomas of the colon and pancreas. U.S. Patent No. 6,492,144 teaches that the Tr-DNA nucleic-acid analysis method may be used to monitor the progress of allogeneic transplants. The presence of transrenal DNA in urine, in the form of nucleic-acid fragments of fewer than 1000 base pairs was also described in Al-Yatama et al. (2001), Prenat Diagn, 21:399-402; and Utting, M., et al. (2002), Clin Cancer Res, 8:35-40. Keiko Koide, et al., Prenat Diagn, 2005; 25: 604-607; Mengjun Wang, et al.., Clinical Chemistry, 2004, 50: 211-213; Y.-H. Su, et al., J. Mol. Diagn., 2004, 6: 101-107.

The presence of transrenal DNA has been explained through the apoptosis phenomenon. During cell death most of the nuclear DNA is converted into nucleosomes and oligomers (Umansky, S.R., et al. 1982, Biochim. Biophys. Acta 655:9-17), which are finally digested by macrophages or neighboring cells. However, a portion of this degraded DNA escapes phagocytic metabolism, and can be found in the bloodstream (Lichtenstein, A.V., et al. 2001, ann NY Acad Scl, 945:239-249), and, as confirmed in the above-indicated patents, also in urine.

The application of this system to pathogenic microorganism infections has never been studied. Previously, it was only known that prokaryotic DNA could be isolated from urine sediment that contained bacteria (Frasier, et al. 1992, Acta Virol, 36:83-89). During a pathogenic infection, prokaryotes and parasites are generally ingested by the cells of the immune system, such as macrophages and dendritic cells. The prokaryotes are then dissolved by the phagolysosome vesicles. The prokaryotic DNA is then released by the cell and a portion of this DNA enters the bloodstream in either of two ways. Either the ingesting cell becomes apoptotic and breaks apart (Navarre, W.V. 2000; Cell Microbiol 2:265-273*);* or the phagolysosome vesicles release the fragments of the prokaryote (including the fragmented DNA) into the bloodstream (Friedlander, A.M. 1978, Infect Immune 22:148-154). However, these fragmented nucleic acids have never been detected in the urine of the infected subject.

The instant invention describes a method of detecting the presence of a non-viral pathogen in a subject through the detection of DNA sequences from the pathogen in the urine of the subject.

### SUMMARY OF THE INVENTION

The invention provides a method for the diagnosis of an infection of a subject through the detection of the presence of a pathogen by the presence of pathogen nucleic acids in a urine sample of the subject. The method of the invention is also used to validate the diagnosis of an infection of a subject through the detection of the presence of pathogen nucleic acids in a urine sample of the subject, wherein a previous diagnosis has already been performed by a method of this invention or another diagnosis method. The method of the invention is also used to determine the efficacy of a treatment of non-viral pathogen infection in a subject by detecting the presence or absence of a nucleic acid of the non-viral pathogen in the urine of the subject. More specifically, the method of the invention is used to identify a drug resistant non-viral pathogenic infection in a subject by detecting the presence or absence of a nucleic acid of the non-viral pathogen in the urine of the subject after the subject has been treated for the infection with a drug. The method of the invention is also used to determine the likelihood of pathology from the infection of the subject by a non-viral pathogen in a subject at risk therefrom. More specifically, the method of the invention is used to genotype a non-viral pathogen which has infected a subject by detecting and genotyping nucleic acids from the pathogen in the urine of the subject.

The invention provides a method for diagnosing a *Mycobacterium tuberculosis* infection in a subject, comprising detecting the presence of a cell-free *Mycobacterium turberculosis* nucleic acid in a soluble fraction of urine sample from the subject, thereby diagnosing the *Mycobacterium tuberculosis* infection. In one embodiment of the method for diagnosing a *Mycobacterium turberculosis* infection in a subject, the method further comprises the step of quantitating the nucleic acid.

In another embodiment of the method for diagnosing a *Mycobacterium turberculosis* infection in a subject, the detecting is performed by a method selected from PCR, nested PCR, semi-nested PCR, hybridization, SSCP, LCR, SDA and pairing with molecular probes that are specific for the non-viral pathogen. In one aspect of this embodiment, a primer set is used for the method, wherein the primer set comprises a forward primer and a reverse primer. Optionally, the forward primer is selected from SEQ ID NOs: 34 and 39 and the reverse primer is selected from SEQ ID NOs: 35, 36 and 40.

In another embodiment of the method for diagnosing a *Mycobacterium turberculosis* infection in a subject, the subject is a mammal. In one aspect of this embodiment, the mammal is human.

### BRIEF DESCRIPTION OF THE DRAWINGS

Figure 1 is a photograph of PCR reactions performed on human urine with primers specific for human b-actin resolved by electrophoresis.
Figure 2 is a photograph ofPCR reactions performed on rabbit urine with primers specific for rabbit b-globin resolved by electrophoresis.

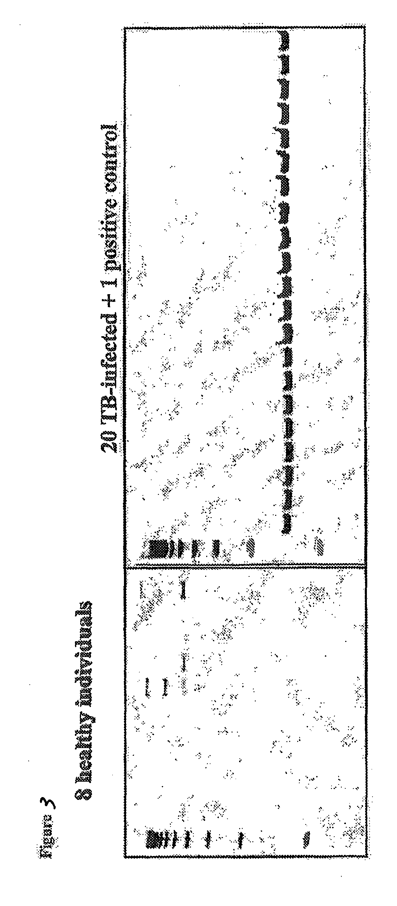

Figure 3 is a photograph of semi-nested PCR reactions performed on human urine from TB infected and healthy patients with primers specific for *Mycobacterium tuberculosis* sequences resolved by electrophoresis.
Figure 4 is a photograph of semi-nested PCR reactions performed on the supernatants and pellets of centrifuged human urine from TB infected and healthy patients before and after treatment with primers specific for *Mycobacterium tuberculosis* sequences resolved by electrophoresis.
Figure 5 shows photographs of semi-nested PCR reactions performed on human urine from TB infected patients with primers specific for *Mycobacterium tuberculosis* sequences of 129/67 bp (left panel) and 330/69 bp (right panel) resolved by electrophoresis.
Figure 6 is a photograph of semi-nested PCR reactions performed on human urine from TB actively infected patients and patients two months after treatment for TB with primers specifc for *Mycobacterium tuberculosis* sequences resolved by electrophoresis.

### DETAILED DESCRIPTION OF THE INVENTION

The invention is based, in part, upon the discovery described herein that pathogenic nucleic acids are detectable in the urine of a subject infected with the pathogen. In some instances, nucleic acids from pathogenic organisms cross the kidney barrier and pass into the urine of mammals. The nucleic acids which cross the renal barrier, termed herein as transrenal nucleic acids, and more specifically transrenal DNA tend to be shorter than 1,000 bp in length, but are preferably less than 500 bp in length, and more preferably shorter than 250-300 bp in length or shorter than 250 bp in length. While it had been shown in the past that DNA from mammalian cells, cancerous and non-cancerous which were hypothesized to die by apoptosis in the body of a subject may be able to cross the renal barrier, this is the first time it has been shown that the DNA of pathogens also crosses this barrier. Further, nucleic acids from pathogens found in the kidney would shed pathogen specific nucleic acids into the urine without crossing the kidney barrier. There may be other mechanisms by which pathogen specific nucleic acids pass into the urine of a subject infected with the pathogen.

Based on this discovery, the invention provides a method for the detection of a non-viral pathogen in the urine of a subject. The method of the invention is used to detect transrenal-nucleic acids derived from a pathogenic microorganism which traverse the renal barrier and are present in the urine of a subject. Described herein are methods for the isolation, amplification and detection of these transrenal nucleic acids.

One method described herein for the isolation of urinary DNA from a subject includes the following steps. Urine is first centrifuged or filtered to separate cells and cell debris from the urine. EDTA/Tris HCl is then added to the urine to bind bivalent ions and to adjust pH to approximately pH 8. To this solution is then added a chaotropic salt, for example guanidine isothiocyanate to a final concentration of at least 3 M. Then a silica resin is added to the urine to which DNA binds. This resin is then washed, and the DNA eluted from it, thereby isolating the transrenal DNA.

DNA isolated from the urine of a subject may then be amplified in order to be detected. Amplification methods include polymerase chain reaction (PCR), nested PCR, semi-nested PCR, Single-Strand Conformation Polymorphism analysis (SSCP), ligase chain reaction (LCR) and strand displacement amplification (SDA). Detection of transrenal DNAs is also performed through hybridization of at least one labeled probe.

The amplification and detection of urinary nucleic acids from a non-viral pathogen may be used to detect the presence of the pathogen, and thus diagnose the infection of a subject with the pathogen and to diagnose pathologies associated with the infection of a subject by the pathogen. Further, the detection of the pathogen may be used to monitor treatment of the infections and pathologies associated with the pathogen. Also, detection of the pathogen may be used to suggest treatment for a subject in which the pathogen is detected. These treatments may be used to relieve symptoms which are associated with the detection of the pathogen or diagnosis with infection of the pathogen.

The bacterial species which is detectable using the method of the invention is *Mycobacterium turberculosis* (*M. turberculosis,* MTB or TB).

### Detection of the Presence of Pathogenic Microorganisms

### Bacteria

### Mycobacterium tuberculosis

Tuberculosis remains the second most common cause of death from an infectious disease in the world in spite of advances in the implementation of control strategies during the last decade (Elzinga G, et al. Lancet 2004;363:814-9). Symptoms of tuberculosis include cough that is worse in the morning (sometimes with hemoptysis, blood in the sputum), chest pain, breathlessness, night sweats, and signs of pneumonia. In advanced disease, there may be extreme weight loss. However, no highly sensitize molecular-based test for the detection of *M*. *tuberculosis* has yet been discovered. Effective treatment of active tuberculosis is the cornerstone of tuberculosis control, and it has been shown that a mere 1% increase in the rate of detection of active cases can save thousands of lives and avert thousand of new tuberculosis cases in high incidence countries (Currie CS, et al. AIDS. 2003;17:2501-8). Consequently, the availability of new diagnostic tools that are more accurate and accessible will greatly benefit individual patients and significantly contribute to the control of the , disease.

Advances in knowledge of the genetic structure of tubercle bacillus have recently contributed to the development of several new molecular methods for detection and identification of *Mycobacterium tuberculosis* from cultures or directly from biological specimens (Drobniewski FA, et al. Lancet Infect Dis. 2003 ;3:141-7). Molecular diagnostic tests which involve the detection of specific nucleic acid sequences could be used for rapid diagnosis of both pulmonary and extra pulmonary tuberculosis. In pulmonary tuberculosis specificity of diagnostic tests based on direct identification of *M*. *tuberculosis* in the sputum using PCR generally exceeds 98% and sensitivity is also high in patients whose sputum smear is positive for acid-fast bacilli on microscopic examination. However, the sensitivity of such tests may be less than 50% for patients with negative sputum smear. (Drobniewski FA, et al., Piersimoni C and Scarparo C. J Clin Microbiol. 2003;41:5355-65; Sarmiento OL, et al. J Clin Microbiol. 2003;41:3233-40). The presence of DNA fragments derived from *M. tuberculosis* have been shown before, but only when the pathogen itself was present in the urine. (Del Portillo et al. J. Clin. Microbiol., 29(10):2163-2168 (1991); Kolk et al. J. Clin Microbiol., 30(10):2567-2575 (1992); Kox et al. Neurology, 45(12):2228-2232 (1995) and Zambardi et al. Mol. Cell. Probes, 9:91-99 (1995)).

The present invention allows for the detection of *M*. *tuberculosis* through identifying its transrenal DNA in the urine of patients with pulmonary tuberculosis. This approach is more attractive than tests using sputum samples because urine specimens are easier and safer to collect than sputum which can generate infectious aerosols and prove to be difficult to obtain, especially in children.

Despite a previous study suggesting the potential utility of a urine test for *M*. *tuberculosis* DNA by PCR in HIV infected patients with pulmonary tuberculosis (Aceti A, et al. Thorax. 1999;54:145-6), subsequent prospective studies conducted on HIV infected and uninfected patients with pulmonary tuberculosis, resulted in only a 40 to 60% sensitivity. This is too low a sensitivity to support the use of a diagnostic test in clinical practice. In these and other such studies, target urinary DNA was of relatively high molecular weight, amplicon sizes of >500 bp having been employed in the PCR reactions. (Kafwabulula M, et al. Int J Tuberc Lung Dis. 2002;6:732-7; Torrea G, et al. J Med Microbiol. 2005;54:39-44).

Previously reported PCR based tests have been used successfully to detect *M*. *tuberculosis* in the urine of patients with genitourinary tuberculosis (van Vollenhoven P, et al. Urol Res. 1996; 24(2):107-11; Hemal AK, et al. Urology. 2000 Oct 1; 56(4):570-4; Moussa OM, et al. J Urol 2000;164:584-8) in which cases the infectious bacteria are present in the urinary tract. Among these patients, high sensitivity and specificity has been achieved by amplifying large (245 to 1000 bp) fragments of mycobacterial DNA purified from urine sediment and the results of molecular test are strongly correlated with the presence of viable mycobacteria which can be cultured from the urine. Thus, presence of specific DNA fragments in these patients appears to reflect urinary excretion of mycobacteria actively replicating in the urogenital tract.

In contrast, we have discovered that small cell-free *M. tuberculosis* DNA fragments originating from sites of infection outside the urogenital tract are present in the urine in this previously unrecognized range of molecular sizes of less than approximately 200 bp. *M*. *tuberculosis* specific DNA sequences can be easily detected as short fragments of less than 200 bp in the soluble fraction of urine specimens from patients with pulmonary tuberculosis. These specific DNA fragments disappear following successful tuberculosis treatment. This is demonstrated in more detail in the Examples below.

The data on *M. tuberculosis* Tr-DNA disappearance during the course of treatment supports the potential utility of this test in monitoring the clinical course of the disease. It is also clear that small urine specimens can be obtained in the field more easily and safely which is important when broad screening of large populations is of concern to public health. It is also likely that Tr-DNA testing for diagnosis of tuberculosis would have particular value for clinics located in regions having limited resources. Although PCR is currently the preferred method for detecting specific nucleic acid sequences, new emerging technologies can be expected to eventually provide the tools to perform the relatively simple Tr-DNA test in the field (Storhoff J.J., et al. (2004) Biosensors & Bioelectronics 19:875-883; Nam J.M., et al. (2002) J. Am. Chem. Soc 124:3820-3821; Ho HA, et al. (2005) J Am Chem Soc. 127:12673-12676) offering practical advantages over those of PCR can be readily applied to the Tr-DNA test. All steps of the diagnostic process, from Tr-DNA purification to biomarker detection, can be automated and brought to the point of care in small hospitals or even in the field with greater facility than can be done with conventional microbiological testing.

### Urinary nucleic acids in non-viral pathogen infections

The present invention describes a method for diagnosing a non-viral pathogen infection by detecting and quantification of the nucleic acids of the pathogenic agent in urine. It has been discovered that the nucleic acids of the pathogenic agent are detectable in urine. Many of these pathogen specific nucleic acids cross the transrenal barrier (Tr-NA) and can be detected in urine as low-molecular-weight fragments (which tend to be shorter than 1,000 bp in length, but are preferably less than 500 bp in length, and more preferably shorter than 250-300 bp in length or shorter than 250 bp in length) through molecular methods known in the art. Other pathogen specific nucleic acids may be shed by cells that are within the kidney, and thus do not have to cross the transrenal barrier in order to be detected in the urine. Futther, some pathogen specific nucleic acids may be found in the urine through other mechanisms besides crossing the transrenal barrier or being generated by cells in the kidney.

The presence of transrenal nucleic acids in the case of infections caused by the pathogenic agent according to the present invention also relates to infections that do not directly involve the urinary tract, even in the absence of hematuria or of pathologies that lead to the rupture, or that alter the normal integrity, of the renal barrier. Transrenal nucleic acids are nucleic acids that have crossed the kidney barrier. Generally, the transrenal nucleic acids (Tr-NA) according to the invention are not associated with, and are not derived from, the DNA of cells that are lost or released in the urinary tract. Instead, the transrenal nucleic acids that are detected in the present invention generally have crossed the kidney barrier as a cell-free material. Thus, their lengths are smaller than about 1000 base pairs, e.g., smaller than about 500, smaller than about 300, smaller than about 250, or between about 100 and about 200 bases or base pairs, as opposed to other cases in which DNA usually has a longer length, greater than 1000 bases or base pairs. In embodiments, the Tr-NAs are DNA fragments.

The discovery confirms the presence of urinary nucleic acids or transrenal nucleic acids derived from pathogenic bacteria and parasites in urine, and therefore is applicable to the diagnosis of all infectious diseases caused by non-viral pathogens.

In one embodiment, the invention relates to a method for diagnosing a non-viral pathogenic infection in a subject by detection of pathogen-related transrenal nucleic acids in a urine sample from the subject. In some embodiments, the invention further includes the step of quantifying the pathogen-related transrenal nucleic acids.

In one embodiment, the invention relates to a method for diagnosis of an infection in a subject, including the step of detection of the presence of pathogen-related transrenal nucleic-acid sequences in a urine sample of the subject. In a further embodiment, the invention relates to the above method in which the transrenal nucleic acids are isolated from the urine sample prior to detecting the nucleic acids.

In another embodiment, the method according to the invention may include an initial treatment of the urine sample prior to detection. In a specific embodiment, the invention includes the pretreatment of the urine sample with agents that inhibit the degradation of the nucleic acids. Included are the enzymatic inhibitors, such as chelating agents, detergents, or denaturing agents, and preferably DNase or RNase inhibitors, which include EDTA, guanidine HCl, guanidine isothiocyanate, N-lauryl sarcosine, and sodium dodecyl sulfate.

According to another embodiment, the urine sample may be centrifuged (at a speed between 3000g and 5000g, such as between 3500g and 4500g) or filtered in order to separate the fraction consisting of cells and their fragments from the supernatant containing the soluble DNA or RNA and their proteinous complexes. However, the urine sample may also be subjected to nucleic acid detection procedures without this fractionation.

The optional isolation or purification and quantification of the transrenal nucleic acids are achieved through the use of chemical or physical methods that are already known in the art. It includes at least one purification step, using methods selected from among extraction with organic solvents, filtration, precipitation, absorption on solid matrices (e.g., via ion exchange), affinity chromatography or else molecular exclusion chromatography or combinations of these methods.

However, the purification method must be appropriate for the isolation of DNA (single- or double-helix) that are less than 1000 nucleotides in length, with a corresponding molecular weight, assuming, as the average molecular weight, that of a nucleotide having a value of 330 Daltons. In some embodiments, the purification is specific for fragments that are smaller than 500 nucleotides (nt) in length, with a corresponding molecular weight, such as fragments whose lengths are less than 300 nt, fragments less than 250 nt in length, or fragments whose lengths are between 100 and 200 base pairs of nucleic acids (nt).

In an embodiment, the DNA isolation method is implemented by pretreating the urine sample with a denaturing agent, such as urea, guanidine HCl, or guanidine isothiocyanate. The sample is then caused to pass through a solid phase, such as a matrix consisting of a silica-based resin which, in the presence of chaotropic salts, such as guanidine isothiocyanate, binds the nucleic acids. Even more preferably, a resin such as Wizard Purification Resin® (Promega^{®}) is utilized. The sample is then collected by elution in a buffer, such as Tris-EDTA (Tris 1-10 mM, EDTA 1-10 mM), or in water.

In a preferred embodiment, the characterization and the determination of the presence of DNA of the pathogenic agent is performed through a technique selected from the group consisting of: hybridization of the nucleic acids, a cycling probe reaction (F. Bekkaoui et al., in BioTechniques 20:240-248 [1996]), a polymerase chain reaction (PCR Protocols: A Guide to Methods and Applications, by M. Innis et al.; Elsevier Publications, 1990), a nested polymerase chain reaction, single-strand conformation polymorphism, or ligase chain reaction (LCR) (F. Barany, in PNAS USA, 88:189-93 [1991]), strand displacement amplification (SDA) (G.K. Terrance Walker, et al., in Nucleic Acid Res, 22:2670-77 [1994], and restriction fragments length polymorphism. Polymerase chain reaction (PCR) is the preferred method for the detection or analysis of nucleic acids. More preferred is dual-amplification PCR, with the use of a nested primer in nested or semi-nested PCR (reference: bio.davidson.edu/courses/genomics/method/NestedPCR.html).

### Methods for the Amplification and Detection of Urinary Nucleic Acids

The term nucleic acid refers to an oligonucleotide, nucleotide, polynucleotide, or fragments/parts thereof and to DNA or RNA of natural (*e.g.*, genomic) or synthetic origin. It may have a double or single helix, and may also represent the sense or antisense direction of a sequence. Parallel helix (5'→3'); antiparallel helix (3'→5'). The terms oligonucleotide, polynucleotide and nucleic-acid polymer are equivalent, and are understood as referring to a molecule consisting of more than two deoxyribonucleic or ribonucleic acid bases. The number of nucleotides (bases) and the length of the oligonucleotide fragment may vary. They may be synthesized in different ways. The sequences are traditionally defined as starting with 5' and ending with a 3'. These numbers indicate the direction of the sequence.

DNA isolated from the urine of a subject may then be amplified in order to be detected. Amplification methods include polymerase chain reaction (PCR), nested PCR, semi-nested PCR, Single-Strand Conformation Polymorphism analysis (SSCP), ligase chain reaction (LCR) and strand displacement amplification (SDA). Detection of transrenal DNAs is also performed through hybridization of at least one labeled primer.

Hybridization is a method that allows two nucleic-acid sequences to recognize each other as complementary and to join together (annealing). Complementarity / Complementary sequences are sequences of polynucleotides that interact with each other, depending on the interaction between the bases. For example, the AGTC sequence is complementary to TCAG according to standard Watson Crick base pairing. However, other combinations such as Hoogstein base pairing are well known to those having ordinary skill in the art. It is possible to have a fully or partially complementary sequence, and this is what determines the efficiency or attractive force between the two sequences. Average complementarity would prevent a strong complementarity from hybridizing, under conditions that would allow it to remain attached.

The ability of nucleic sequences to hybridize is a well-known phenomenon. The first hybridization method was described in Marmur & Lane, PNAS USA, 46:453 (1960) and 461 (1960), but since then has been perfected as a technique in molecular biology. Today, the term "hybridization" includes, among others, slot/dot and blot hybridization. The conditions that allow nucleotide sequences to recognize each other (hybridization) can be modified in such a way as to produce complete hybridization (complementarity with high specificity) or partial hybridization (complementarity with average specificity). In the present application, whenever the term "hybridization" is used, the conditions should be understood as referring to those that allow average or high complementarity. The technician in the field can calculate how many artificial sequences are needed to encourage hybridization between two complementary sequences in the opposite direction, known as antiparallel association.

A probe is an oligonucleotide that can be produced artificially or naturally, and that forms a combination with another nucleic-acid sequence. The probes are useful in discovering specific sequences in a sample containing unknown DNA. In this patent, all of the probes can be bound to a signaling molecule (or reporter). The reporter molecule makes it possible to detect the probe (for example, through enzymatic reactions (*e.g*., ELISA (Enzyme-Linked Immunosorbent Assay)), radioactivity, fluorescence, or other systems).

Polymerase chain reaction (PCR) is a method of amplification of a DNA sequence using complementary primers and a heat sensitive polymerase. One class of enzymes utilized in the amplification of specific nucleic acids are DNA polymerases referred to as Taq (*Thermus aquaticus*) polymerases. Primers are oligonucleotides from which, under proper conditions, the synthesis of a polynucleotide sequence can be initiated. A primer may exist naturally (for example, in an enzymatic digestion of a polynucleotide), or may be obtained through chemical synthesis. The product amplified in PCR is often referred to as an amplicon.

Nested PCR is a second PCR which is performed on the product of an earlier PCR using a second set of primers which are internal to the first set of primers, referred to as nested primers. This significantly improves the sensitivity and specificity of the PCR. Nested primers are primers internal to an amplicon obtained with a first PCR cycle. The amplification process that uses at least one nested primer improves specificity, because the non-specific products of the first cycle are not amplified in the second cycle, because they lack the sequence that corresponds to the nested primer. Semi-nested PCR is a second PCR which uses one new primer and one of the original primers. This process also improves specificity.

Ligase Chain Reaction (LCR) is a method of DNA amplification similar to PCR. LCR differs from PCR because it amplifies the probe molecule rather than producing an amplicon through polymerization of nucleotides. Two probes are used per each DNA strand and are ligated together to form a single probe. LCR uses both a DNA polymerase enzyme and a DNA ligase enzyme to drive the reaction. Like PCR, LCR requires a thermal cycler to drive the reaction and each cycle results in a doubling of the target nucleic acid molecule. LCR can have greater specificity than PCR.

In Single-Strand Conformation Polymorphism (SSCP) analysis a small PCR product (amplicon) is denatured and electrophoresed through a non-denaturing polyacrylamide gel. Thus, as the PCR product moves into and through the gel (and away from the denaturant), it will regain secondary structure that is sequence dependent (similar to RNA secondary structure). The mobility of the single-stranded PCR products will depend upon their secondary structure. Therefore, PCR products that contain substitutional sequence differences as well as insertions and deletions will have different mobilities.

Strand displacement amplification (SDA) is an isothermal nucleic acid amplification method based on the primer-directed nicking activity of a restriction enzyme and the strand displacement activity of an exonuclease-deficient polymerase.

The terms purification or isolation refers to a process for removing contaminants from a sample, where the result is a sample containing 50%, 60%, 75%, 90% or over 90% of the material toward which the purification procedure is directed.

For stringent temperature conditions in the case of nucleic-acid hybridization, these terms usually refer to a variable temperature between a maximum, for a nucleic acid, represented by Tm less 5°C., and a minimum represented by Tm less 25°C. The technique used in the field utilizes stringent temperature conditions, in combination with other parameters (*e*.*g*., saline concentration), to distinguish sequences with a quasi-exact homology.

Stringent conditions are known to those skilled in the art and can be found in Ausubel, et al., (eds.), CURRENT PROTOCOLS IN MOLECULAR BIOLOGY, John Wiley & Sons, N.Y. (1989), 6.3.1-6.3.6. Preferably, the conditions are such that sequences at least about 65%, 70%, 75%, 85%, 90%, 95%, 98%, or 99% homologous to each other typically remain hybridized to each other. A non-limiting example of stringent hybridization conditions are hybridization in a high salt buffer comprising 6X SSC, 50 mM Tris-HCl (pH 7,5), 1 mM EDTA, 0.02% PVP, 0.02% Ficoll, 0.02% BSA, and 500 mg/ml denatured salmon sperm DNA at 65°C, followed by one or more washes in 0.2X SSC, 0.01% BSA at 50°C.

In a second embodiment, a nucleic acid sequence that is hybridizable to the nucleic acid molecule comprising the nucleotide sequence, or fragments, analogs or derivatives thereof, under conditions of moderate stringency is used. A non-limiting example of moderate stringency hybridization conditions are hybridization in 6X SSC, 5X Reinhardt's solution, 0.5% SDS and 100 mg/ml denatured salmon sperm DNA, at 55 °C, followed by one or more washes in 1X SSC, 0.1% SDS at 37°C. Other conditions of moderate stringency that may be used are well-known within the art. *See, e.g.,* Ausubel, et al. (eds.), 1993, CURRENT PROTOCOLS IN MOLECULAR BIOLOGY, John Wiley & Sons, NY, and Krieger, 1990; GENE TRANSFER AND EXPRESSION, A LABORATORY MANUAL, Stockton Press, NY.

In a third embodiment, a nucleic acid that is hybridizable to the nucleic acid molecule, or fragments, analogs or derivatives thereof, under conditions of low stringency, is used. A non-limiting example of low stringency hybridization conditions are hybridization in 35% formamide, 5X SSC, 50 mM Tris-HCl (pH 7.5), 5 mM EDTA, 0.02% PVP, 0.02% Ficoll, 0.2% BSA, 100 mg/ml denatured salmon sperm DNA, 10% (wt/vol) dextran sulfate at 40°C, followed by one or more washes in 2X SSC, 25 mM Tris-HCl (pH 7,4), mM EDTA, and 0.1% SDS at 50°C. Other conditions of low stringency that may be used are well known in the art (*e.g.,* as employed for cross-species hybridizations). *See, e.g.,* Ausubel, et al. (eds.), 1993, CURRENT PROTOCOLS IN MOLECULAR BIOLOGY, John Wiley & Sons, NY, and Kriegler, 1990, GENE TRANSFER AND EXPRESSION, A LABORATORY MANUAL, Stockton Press, NY; Shilo and Weinberg, 1981. Proc Natl Acad Sci USA 78: 6789-6792.

Described herein is a kit for detecting and/or genotyping *Mycobacterium tuberculosis* in a urine sample from a subject in need thereof, comprising at least one forward primer selected from SEQ ID NOs: 34 and 39 and at least one reverse primer selected from SEQ ID NOs: 35, 36 and 40, either in the same or separate packaging, and instructions for its use.

The invention will be further described in the following examples, which do not limit the scope of the invention described in the claims.

### EXAMPLES

All of the methodology described herein may be modified by the technician in the field with no change in the basic principal idea.

### Example 1. Stabilization and preparation of the samples.

All of the steps of the preparation of the urine samples and of the analysis of the transrenal DNA were performed at room temperature. Briefly, approximately 50-60 ml of urine samples were collected from each patient participating in the study. Within 30 minutes after collection, a solution consisting of 0.5M EDTA and 0.5M Tris-HCl, at a pH ranging from 8.0 to 8.5, was added at a final concentration of 10 mM in order to inhibit the nucleases that might be present in urine samples. At least three nucleolytic enzymes were identified in urine, DNase I, DNase II and phosphodiesterase. EDTA inhibits the activity of DNase I and phosphodiesterase by chelating divalent metals such as Mg²⁺, Ca²⁺ and/or others that are required for their activity. DNase II is maximally active in acidic environment. Supplementing the urine with Tris-HCl pH 8.0 buffer increases the pH of urine thereby inhibiting the activity of DNase II.

The stabilized urine samples can be stored, in aliquots of 5 ml, at -80°C. Optionally, 25 mL of saline solution is frozen to act as a control.

Other methods for the preparation of urine samples and for the extraction of the DNA are described in PCT patent No. WO 98/54364, which is incorporated by reference in its entirety.

### Example 2. Urine fractionation.

For Tr-DNA based applications, that involve quantitative Tr-DNA analysis, urine fractionation is often required. It reduces the impact of nucleoproteins from cells present in urine on accurate quantitation of Tr-DNA/protein complex, total Tr-DNA, or specific genetic markers. In our experiments we have employed two procedures for urine fractionation, centrifugation and filtration. Both were carried out immediately after urine collection and, before supplementing the specimen with EDTA-Tris stabilizing mixture. Urine may be kept in this condition as long as urine cells are intact and their constituents do not leak into urine and contaminate Tr-DNA by cellular DNA.

For filtration one can use fiiters with pore size ranging from 0.1 to 5 µm with reduced nucleic acid and protein binding capacity. The choice of the filter depends on target under analysis. For bacterial pathogens smaller pores are required. In our experiments we used luer-lock 0.45 µm Filter Units (Cat. No. SLHV033RS) or 0.45 µm 150 ml Filter Unit STERICAP (Cat. No. SCHVU01RE) both from Millipore. After filtration the samples were supplemented with EDTA-Tris conservation solution and taken into downstream processes of DNA extraction or aliquoted and stored frozen at -80 °C. Urine cellular fraction can be harvested by extracting it from the filter by applying a solution (high concentrations of salts or chaotropic agents) that is known to dissolve cells.

Urine fractionation by centrifugation in our experiments was carried immediately after sample collection, before addition of preservation mixture. Centrifugation was performed at room temperature at RCF ranging between 3500 and 4000xg for approximately 15 min. Supernatant was carefully collected and manipulated per the procedure described above for urine filtration. The pellet consisting mainly of cells and cellular debris was resuspended in physiological solution and stored at -80 °C.

### Example 3. Extraction and purification of the nucleic acids from urine.

In our experiment we used silica based DNA extraction protocol in two different formats. One was vacuum driven and the second, centrifugation.

### Vacuum Based Procedure

To the urine sample 6 M Guanidine Thiocyanate (GITC) (AMRESCO, Cat. No.0380) was added to final concentration not less than 3 M. This solution was mixed vigorously and supplemented with 0.25 ml of Wizard silica suspension (PROMEGA, Cat. No. A7141) per 10 ml of initial urine volume. To capture DNA the mixture was continuously rotated for 1 hour at room temperature. Resin with captured DNA was collected by loading the mixture on a minicolumn (PROMEGA, Cat. No. A7211) syringe assembly attached to a vacuum line. Resin was collected by applying vacuum, washed twice with 5 ml of 3M GITC solution. The resin was further washed twice by a solution consisting of 80% ethanol and 50 mM NaCl. Then the minicolumn was detached and the resin was additionally washed twice with 96% ethanol by centrifugation on a benchtop microcentrifuge in 1.5 ml tubes. DNA was eluted by brief centrifugation with hot water in a volume of not more than 1/20 of urine initial volume.

### Centrifugation Based Procedure

To the urine sample 6 M Guanidine Thiocyanate (GITC) (AMRESCO, Cat. No.0380) was added to final concentration not less than 3 M. This solution was mixed vigorously and supplemented with 0.25 ml of Wizard silica suspension (PROMEGA, Cat. No. A7141) per 10 ml of initial urine volume. To capture the DNA, the mixture was continuously rotated for 1 hour at room temperature. Resin with captured DNA was collected by centrifugation at approximately 4000xg for about 5 min at room temperature. Pelleted resin was washed once with GITC solution and once with wash buffer, then was transferred into a mini-column and washed one additional time with 96% ethanol. DNA was eluted with hot water into a microcentrifuge tube by brief centrifugation. DNA was eluted by brief centrifugation with hot water in a volume of not more than 1/20 of urine initial volmne.

### Example 4. Monitoring of the integrity of DNA extraction procedure.

The DNA extraction procedure was routinely monitored by testing the purified DNA for the presence of selected control DNA sequences. For the purposes of detection of Tr-DNA of infectious agents the integrity of DNA purification procedure was monitored using beta-actin and beta-globin as markers for human and rabbit urine, respectively. PCR amplification of above targets is a surrogate for the estimation of DNA yield and quality. Primer pairs used in these studies are presented in Table 1.

**Table 1.**

| **ID** | **Sequence** | **Pr. Length (nt)** | **Tm** | **Amp. Size (bp)** | **SEQ ID NO:** |
|---|---|---|---|---|---|
| ES0007 | AAGACAGTGTTGTGGGTGTAGG | 22 | 59.0 | 77 | 1 |
| ES0008 | TCCAAGGCCGCTTTACAC | 18 | 59.8 | | 2 |
| LEBG_F | GGCACAGGTTTCATCCATTC | 20 | 54.1 | 70 | 3 |
| LEBG_R | GATAGGTCTCTCTTTATGTCTTCAG | 25 | 54.4 | | 4 |

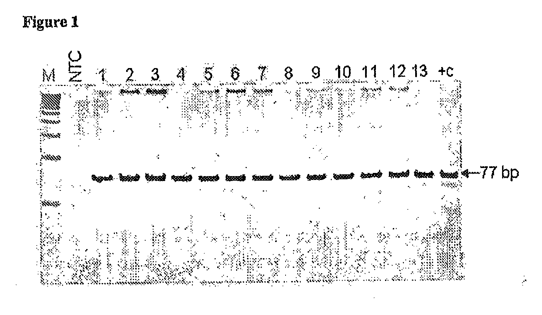

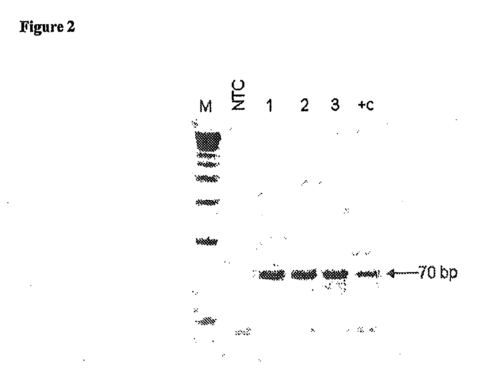

PCR conditions for both targets were identical. Amplification of isolated DNA equivalent to 300 µl of urine was carried out in 25 µl mixture containing 0.2 µmol/liter of primers ES0007 and ES0008 or LEBG_F and LEBG_R for human b-actin or rabbit b-globin, respectively, 200 µmol/liter dNTP each, and 1 U GoTaq Polymerase (Promega). Thermal profile for both types of reactions was: 94 °C for 5 minutes followed by 35 cycles of 94 °C for 30 seconds, 60 °C for 30 seconds, 72 °C for 1 minute, and 1 cycle of 72 °C for 5 minutes. Ten µl the products were resolved by electrophoresis in 7% polyacrylamide gel, and visualized by ethidium bromide staining. Figure 1 and Figure 2 depict the results of PCR of DNA purified from human urine and rabbit urine, respectively.

Figure 1 shows the results of PCR performed to control the integrity of DNA extraction from human urine. Urine samples are from patients attending doctor's office for endogastroscopy for a test for *Helicobacter pylori.* M - DNA molecular weight standards, 50 bp ladder; NTC - no template control; 1 - 13 urine from patients.

Figure 2. shows the results of PCR performed to control the integrity of DNA extraction from rabbit urine. Urine samples were harvested from rabbits infected with *Bacillus anthracis* spores. M - DNA molecular weight standards, 50 bp ladder; NTC - no template control; 1 - DNA from urine of uninfected rabbit; 2 and 3 - urine of infected rabbit collected 6 an 14 hours after infection, respectively; +c - rabbit genomics DNA

### Example 5. Design of PCR primers.

As a general rule, primers for the PCR based analysis of Tr-DNA were designed for target amplicons not more than 200 bp in length.

All primers were screened against the complete sequence of human genome.

For primer design FastPCR software was used (http://www.biocenter.helsinki.fi/bi/bare-1_html/oligos.htm). The critical parameters for primer design were as follows:
- Allow 1 mismatch in 5 nucleotides at 3' end.
- At least 2 G or C residues at 3' end.
- Only one A or T residue at 3' end.
- Maximum Tm of 23 °C for 6 nucleotides at 3' end.

Nested primers were designed using Primer 3 package [see Steve Rozen and Helen J. Skaletsky (2000) Primer3 on the WWW for general users and for biologist programmers. In: Krawetz S, Misener S (eds) Bioinformatics Methods and Protocols: Methods in Molecular Biology. Humana Press, Totowa, NJ, pp 365-386] and is available online (http://frodo.wi.mit.edu/cgi-bin/primer3/primer3_www.cgi). The only parameter specified for the nested primers was the Tm in order to have primers with annealing temperature not less than external primers.

### Example 6. Selection of PCR. Primer Sets for the Detection of Mycobacterium tuberculosis.

PCR primers for the detection of *M. tuberculosis* complex species DNA were selected for the region of IS6110 shown to be highly specific (Hellyer). Primers used in this study are presented in Table 2.

**Table 2.**

| **ID** | **Sequence** | **Product size, bp** |
|---|---|---|
| F-785 | ACCAGCACCTAACCGGCTGTGG (SEQ ID NO: 34) | 129 |
| R-913 | CATCGTGGAAGCGACCCGCCAG (SEQ ID NO: 35) | |
| Rn-851 | GTAGGCGAACCCTGCCCAGGTC (SEQ ID NO: 36) | 67 |
| F-489 | GCCCCATCGACCTACTACTACG (SEQ ID NO: 37) | 330 |
| R-819 | TGAGGTCTGCTACCCACAGC (SEQ ID NO: 38) | |
| Fn-627 | CCCTGAACCGTGAGGGCATGG (SEQ ID NO: 39) | 69 |
| Rn-690 | ACAGGCCGAGTTTGGTCATCAGC (SEQ ID NO :40) | |

### Example 7. Detection of Mycobacterium tuberculosis bacteria DNA sequences in urine

All study participants were enrolled at the National Institute for Infectious Diseases (INMI) "L. Spallanzani" in Rome, Italy. In this study we included patients who had been clinically diagnosed with pulmonary tuberculosis as confirmed by isolation of *M. tuberculosis* from sputum culture. Nineteen of the 20 patients had a sputum smear positive for acid-fast bacilli, whereas, only one was HIV positive. No patient exhibited clinical evidence of extra-pulmonary involvement, and all urine cultures for *M. tuberculosis* performed were negative. Urine specimens were obtained either before or within one week of initiation of anti-tuberculosis therapy. Information regarding age, sex, ethnicity, *M. bovis* BCG vaccination, and results of laboratory examinations for tuberculosis were collected. In order to determine whether subsequent therapy had an influence on the ability to detect M *tuberculosis* DNA, 8 of the patients with pulmonary tuberculosis were asked to return to donate urine specimens two months after initiating anti-tuberculosis therapy. In addition, ten healthy individuals were included as controls in the study.

Urine specimens were processed as described in Examples 1-3 above.

Twenty cycles of PCR amplification were performed as following: isolated DNA equivalent to that contained in 300 µl of urine was added to a 25 µl mixture containing 0.2 µmol/liter of outer primers F-785 and R-913, 5 µl 5X PCR buffer (Promega), 200 µmol/liter dNTP each, and 1 U GoTaq Polymerase (Promega), denatured at 94 °C for 5 minutes followed by 20 cycles of 94 °C for 30 seconds, 62 °C for 30 seconds, 72 °C for 1 minute, and 1 cycle of 72 °C for 5 minutes. 1 µl of the product from this amplification was diluted 1:10 and 1 µl of the dilution was re-amplified 35 cycles using primers F-785 and Rn-851 under the same conditions as in the first reaction. Identical setting was used for the amplification of large, 330 bp fragment. The products from the second amplification were resolved by electrophoresis in 7% polyacrylamide gel, and visualized by ethidium bromide staining.

Results from semi-nested amplification are shown in the right panel of Figure 2. The *M. tuberculosis*-specific product of 67 bp was found in urine specimens from all 20 patients analyzed. Eight healthy individuals used as controls were all found to be negative for the presence of *M. tuberculosis*-specific sequences. The last lane in the gels is the genomic DNA of Mycobacterium tuberculosis strain H37RV, as a positive control.

### Example 8. Demonstration that TB specific DNA sequences found in urine are cell free.

Previous reports on detection of *M. tuberculosis* DNA in urine employed isolation methods designed to extract high molecular weight DNA from whole urine or urinary sediments. Furthermore, in these reports larger amplicon sizes were used (>200 bp) (Torrea G. J., et al. Med Microbiol. 2005;54:39-44; Piersimoni C. J., et al. Clin. Microbial. 40:4138-4142 (2002) Kafwabulula M., et al. Int J Tuberc Lung Dis. 2002;6:732-7; Marei A.M., et al. J Med Microbiol. 2003 Apr;52(Pt 4):331-5. Del Portillo P. et al. J Clin Microbiol. 1991 Oct;29(10):2163-8). Therefore, it was important to further characterize the nature of the *M. tuberculosis.* Tr-DNA found in urine. In order to address this question, two experimental designs were employed. In the first, specimens were fractionated by centrifuged and DNA was isolated separately from urine sediment and supernatant (see Examples 2 and 3).

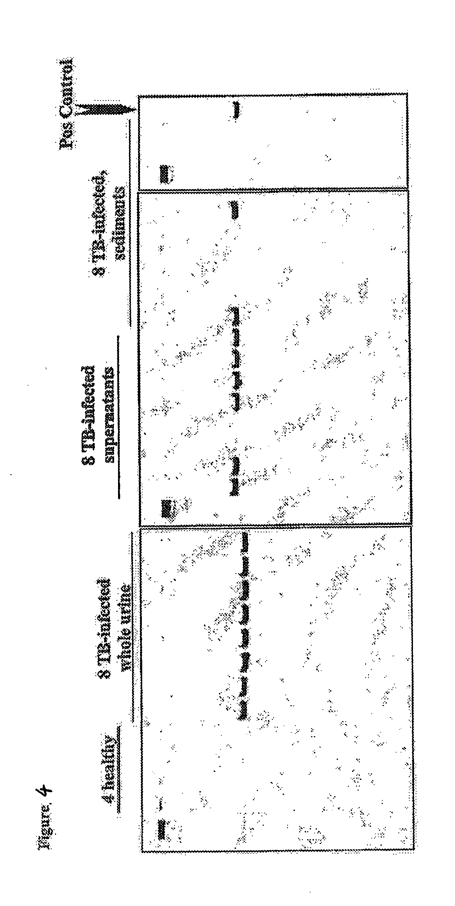

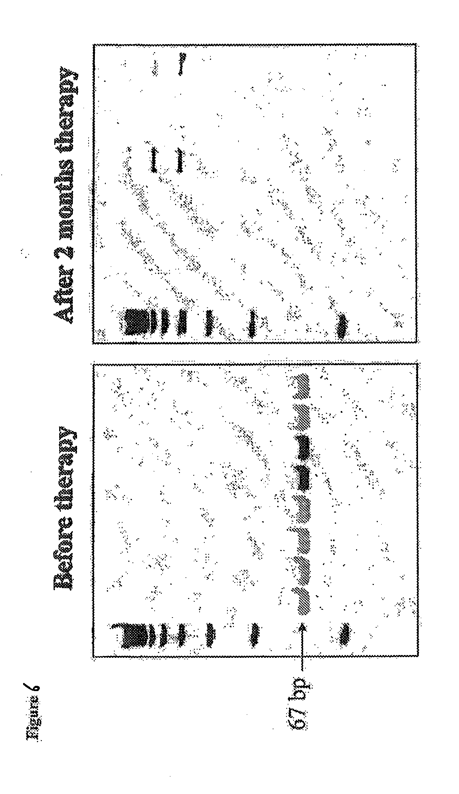

Primer pairs F-785/R-913 and semi-nested primers F-785/Rn-851 specific for short amplicon were used (see Table 2). As shown in Figure 4, 6 of 8 of the supernatant samples display the presence of *M. tuberculosis* DNA, whereas only 2 of 8 of the pellet samples were positive.

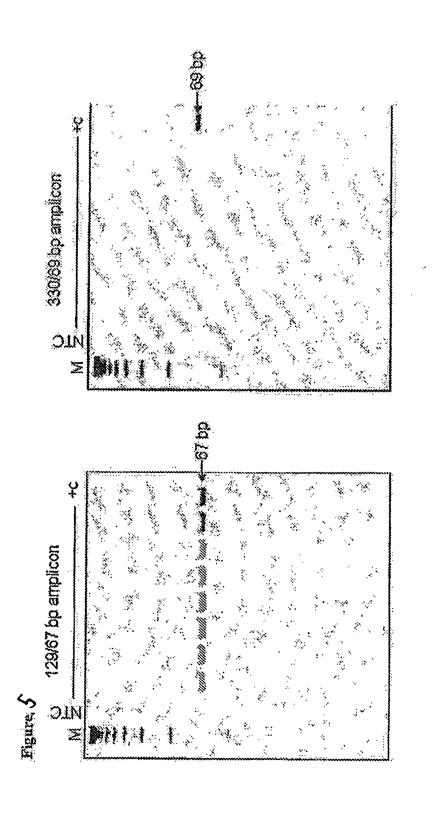

The second experiment was aimed on comparison of PCR products obtained with primers designed for larger amplicon size, in the range used by several other investigators. Results shown in Figure 5 demonstrate that MTB DNA sequences again were detected in all 7 samples of DNA that had been isolated from urine of pulmonary TB patients when using primers for 129/67 amplicons. However, no PCR products were detected with primers for 330/69 amplicons. In Panel A semi-nested PCR for the short amplicon (target 129 bp). In Panel B semi-nested PCR for the long amplicon with primers F489/R819 (target 330 bp). Respective final semi-nested PCR products for short and long amplicons are 67 bp and 69 bp. Lanes 3-9 represent products of PCR amplification of DNA purified from patients urine. Lanes 1 in both gels show the DNA standards, Lanes 2 in both gels are negative controls, and Lanes 10 in both gels are positive genomic DNA controls. These data confirmed that bacterial DNA fragments extracted from urine are relatively short fragment. Furthermore, these results strongly suggest that it is unlikely that MTB DNA can be reproducibly detected in urine specimens of pulmonary TB patients when using urine sediment PCR analysis combined with large amplicon sizes.

### Example 9. Analysis of TB Patients Before and After Treatment.

One of the critical problems faced in the management and eradication of pulmonary tuberculosis worldwide is the accurate and early detection of the efficacy of therapeutic treatment of patients. Currently general practice of monitoring of the success of a treatment is based'on clinical symptoms, which are relatively late indicators and therefore do not provide information for a "real time" adjustment of the regiment of therapy.

In this regard the impact of Tr-DNA platform technology is twofold. First, it gives a safe, easy and inexpensive means for detection of mutations known to be associated with increased drug resistance, and second, it ideally fits to the early monitoring tasks owing to its inherent capabilities of detection and quantification of non-host origin genetic markers in urine.

In order to determine whether MTB-specific DNA sequences in urine can be used for evaluation of therapeutic treatment, 8 of the patients with TB who had been found to be MTB Tr-DNA positive at enrolment were asked to return to donate urine samples approximately two months following initiation of chemotherapy. At the time of the second test clinical symptoms have resolved in these patients and all were sputum smear negative. Tr-DNA extracted from these samples was analyzed and no product corresponding to 67 bp specific TB sequence was observed, as shown in Figure 6.

## Claims

1. A method for diagnosing a *Mycobacteriutm tuberculosis* infection in a subject, comprising detecting the presence of a cell free *Mycobacterium tuberculosis* nucleic acid in a soluble fraction of urine sample from the subject, thereby diagnosing the *Mycobacterium tuberculosis* infection.

2. The method of claim 1, further comprising the step of quantitating the nucleic acid.

3. The method of claim 2, wherein said detecting is performed by a method selected from the group consisting of PCR, nested PCR, semi-nested PCR, hybridization, SSCP, LCR, SDA, and pairing with molecular probes that are specific for the non- viral pathogen.

4. The method of claim 3, wherein a primer set is used for said method, wherein the primer set comprises a forward primer and a reverse primer.

5. The method of claim 4, wherein the forward primer is selected from the group consisting of SEQ ID NOs: 34 and 39.

6. The method of claim 4, wherein the reverse primer is selected from the group consisting of SEQ ID NOs: 35, 36, and 40.

7. The method of claim 1, wherein the subject is a mammal.

8. The method of claim 7, wherein the mammal is a human.

9. The method of any preceding claim wherein the nucleic acid detected is one or more DNA fragments of less than 300bp.

10. The method of any preceding claim wherein the nucleic acid detected is one or more DNA fragments of 200bp or less.

## Patentansprüche

1. Verfahren zur Diagnose einer *Mycobacterium-tuberculosis-*Infektion bei einem Patienten, umfassend das Detektieren des Vorhandenseins einer zellfreien *Mycobacterium-tuberculosis-*Nucleinsäure in einer löslichen Fraktion einer Urinprobe von dem Patienten, wodurch die *Mycobacterium-tuberculosis*-Infektion diagnostiziert wird.

2. Verfahren nach Anspruch 1, des Weiteren umfassend eine Stufe der Quantifizierung der Nucleinsäure.

3. Verfahren nach Anspruch 2, wobei das Detektieren durchgeführt wird durch ein Verfahren, ausgewählt aus der Gruppe, bestehend aus PCR, verschachtelte PCR, halbverschachtelte PCR, Hybridisierung, SSCP, LCR, SDA, und Paarung mit molekularen Proben, die für das nicht-virale Pathogen spezifisch sind.

4. Verfahren nach Anspruch 3, wobei ein Primerset für das Verfahren verwendet wird, wobei das Primerset einen Forward-Primer und einen Reverse-Primer umfasst.

5. Verfahren nach Anspruch 4, wobei der Forward-Primer ausgewählt ist aus der Gruppe, bestehend aus SEQ ID NOs: 34 und 39.

6. Verfahren nach Anspruch 4, wobei der Reverse-Primer ausgewählt ist aus der Gruppe, bestehend aus SEQ ID NOs: 35, 36 und 40.

7. Verfahren nach Anspruch 1, wobei der Patient ein Säugetier ist.

8. Verfahren nach Anspruch 7, wobei das Säugetier ein Mensch ist.

9. Verfahren nach einem der voranstehenden Ansprüche, wobei die detektierte Nucleinsäure ein oder mehr DNA-Fragmente von weniger als 300 bp ist bzw. sind.

10. Verfahren nach einem der voranstehenden Ansprüche, wobei die detektierte Nucleinsäure ein oder mehr DNA-Fragmente von 200 bp oder weniger ist bzw. sind.

## Revendications

1. Procédé permettant de diagnostiquer chez un sujet une infection par *Mycobacterium tuberculosis*, lequel procédé comporte le fait de détecter la présence d'un acide nucléique de *Mycobacterium tuberculosis,* sans cellules, dans une fraction soluble d'un échantillon d'urine du sujet, ce qui permet de diagnostiquer une infection par *Mycobacterium tuberculosis.*

2. Procédé conforme à la revendication 1, qui comporte en outre une étape de détermination de la quantité dudit acide nucléique.

3. Procédé conforme à la revendication 2, dans lequel ladite détection est opérée par une méthode choisie dans l'ensemble formé par les PCR (réaction en chaîne de polymérase), PCR nichée, PCR semi-nichée, hybridation, SSCP (analyse de polymorphisme de conformation de simple brin), LCR (réaction en chaîne de ligase), SDA (amplification avec déplacement de brin), et appariement avec des sondes moléculaires spécifiques des agents pathogènes non-viraux.

4. Procédé conforme à la revendication 3, dans lequel on utilise pour ladite méthode un jeu d'amorces, lequel jeu d'amorces comprend une amorce sens et une amorce anti-sens.

5. Procédé conforme à la revendication 4, pour lequel l'amorce sens est choisie dans l'ensemble formé par les Séquences N° 34 et N° 39.

6. Procédé conforme à la revendication 4, pour lequel l'amorce anti-sens est choisie dans l'ensemble formé par les Séquences N° 35, N° 36 et N° 40.

7. Procédé conforme à la revendication 1, dans lequel le sujet est un mammifère.

8. Procédé conforme à la revendication 7, dans lequel le mammifère est un humain.

9. Procédé conforme à l'une des revendications précédentes, dans lequel l'acide nucléique détecté est un ou plusieurs fragment(s) d'ADN long(s) de moins de 300 paires de bases.

10. Procédé conforme à l'une des revendications précédentes, dans lequel l'acide nucléique détecté est un ou plusieurs fragment(s) d'ADN long(s) de 200 paires de bases ou moins.
